# EUROPEAN PATENT APPLICATION

(11) **EP 4 699 565 A2**
(43) Date of publication of application: **25.02.2026**
(21) Application number: 26151586.0
(22) Date of filing: 28.07.2022
(51) Int. Cl.: A61B 34/30

(54) **GAS SEALED ACCESS CAP FOR A ROBOTIC CANNULA**

(30) Priority: 30.07.2021 US 202163227449 P; 10.08.2021 US 202163231390 P
(62) Divisional of application: 22850300.9
(71) Applicant: CONMED Corporation, Largo, FL 33773 (US)
(72) Inventor: THOMPSON, Emily, Castle Rock, CO, 80104 (US); BRENNER, Corey, London, Littleton, CO, 80123 (US)
(74) Representative: Hoefer & Partner Patentanwälte mbB

(57) **Abstract**

. A gas sealed access cap for a robotic cannula is disclosed which includes a housing defining an interior cavity that supports an annular jet assembly for receiving pressurized gas from an inlet port of the housing, wherein the annular jet assembly is adapted to generate a gaseous sealing zone within the robotic cannula to maintain a stable pressure within the surgical cavity of a patient during the performance of a robotically assisted laparoscopic surgical procedure.

## Description

### CROSS-REFERENCE TO RELATED APPLICATIONS

This subject application claims the benefit of priority to U.S. Provisional Patent Application No. 63/227,449, filed July 30, 2021 and U.S. Provisional Patent Application No. 63/231,390, filed August 10, 2021, the disclosures of which are incorporated herein by reference in their entireties, including all appendixes thereof.

### BACKGROUND OF THE DISCLOSURE

### 1. Field of the Disclosure

The subject disclosure is directed to endoscopic surgery, and more particularly, to a gas sealed access cap for performing robotically assisted laparoscopic surgical procedures using a robotic cannula, and a method of using the same.

### 2. Description of Related Art

Laparoscopic or "minimally invasive" surgical techniques are becoming commonplace in the performance of procedures such as cholecystectomies, appendectomies, hernia repair and nephrectomies. Benefits of such procedures include reduced trauma to the patient, reduced opportunity for infection, and decreased recovery time. Such procedures within the abdominal (peritoneal) cavity are typically performed through a device known as a trocar or cannula, which facilitates the introduction of laparoscopic instruments into the abdominal cavity of a patient.

Additionally, such procedures commonly involve filling or "insufflating" the abdominal cavity with a pressurized fluid, such as carbon dioxide, to create an operating space, which is referred to as a pneumoperitoneum. The insufflation can be carried out by a surgical access device, such as a trocar, equipped to deliver insufflation fluid, or by a separate insufflation device, such as an insufflation (Veress) needle. Introduction of surgical instruments into the pneumoperitoneum without a substantial loss of insufflation gas is desirable, in order to maintain the pneumoperitoneum.

During typical laparoscopic procedures, a surgeon makes three to four small incisions, usually no larger than about twelve millimeters each, which are made with the surgical access devices themselves, often using a separate inserter or obturator placed therein. Following insertion, the obturator is removed, and the trocar allows access for instruments to be inserted into the abdominal cavity. Typical trocars provide a pathway to insufflate the abdominal cavity, so that the surgeon has an open interior space in which to work.

The trocar must also provide a way to maintain the pressure within the cavity by sealing between the trocar and the surgical instrument being used, while still allowing at least a minimum amount of freedom of movement for the surgical instruments. Such instruments can include, for example, scissors, grasping instruments, and occluding instruments, cauterizing units, cameras, light sources and other surgical instruments. Sealing elements or mechanisms are typically provided on trocars to prevent the escape of insufflation gas from the abdominal cavity. These sealing mechanisms often comprise a duckbill-type valve made of a relatively pliable material, to seal around an outer surface of surgical instruments passing through the trocar.

SurgiQuest, Inc., a wholly owned subsidiary of ConMed Corporation has developed unique gas sealed surgical access devices that permit ready access to an insufflated surgical cavity without the need for conventional mechanical valve seals, as described, for example, in U.S. Pat. No. 8,795,223. These devices are constructed from several nested components including an inner tubular body portion and a coaxial outer tubular body portion. The inner tubular body portion defines a central lumen for introducing conventional laparoscopic surgical instruments to the abdominal cavity of a patient and the outer tubular body portion defines an annular lumen surrounding the inner tubular body portion for delivering insufflation gas to the abdominal cavity of the patient and for facilitating periodic sensing of abdominal pressure.

Robotically assisted minimally invasive surgical procedures have also become increasingly more common. One well-known system for performing these procedures is called the Da Vinci robotic surgical system, which is manufactured and sold by Intuitive Surgical, Inc. of Sunnyvale, Calif. The Da Vinci system utilizes a proprietary trocar or cannula that is adapted and configured to receive robotic instruments and be engaged by a robotic arm. The proprietary Da Vinci cannula has a proximal housing that forms a bowl for receiving components such as a gas-tight seal assembly, as disclosed for example in U.S. Pat. No. 10,463,395. The Da Vinci gas-tight seal assembly utilizes mechanical seals to seal around an outer surface of surgical instruments passing through the cannula and to prevent the escape of insufflation gas from the abdominal cavity.

It is believed to be beneficial to provide a seal assembly for use with the Da Vinci cannula that permits ready access to an insufflated surgical cavity without the need for a mechanical seal assembly. Indeed, a recent example of such a valve-less seal assembly is disclosed in commonly assigned U.S. Patent No. 11,026,717, which describes a gas sealed access cap for use with the Da Vinci robotic cannula. The subject disclosure improves upon this earlier gas sealed access cap and provides a useful method of installing and using the access cap with a robotic cannula to perform a laparoscopic surgical procedure.

### SUMMARY OF THE DISCLOSURE

The subject disclosure is directed to a new and useful gas sealed access cap for use with a robotic cannula, which provides stable pneumoperitoneum for constant exposure, even during suction and leaks, enables surgeons to confidently operate at low intra-abdominal pressure, provides constant smoke evacuation to ensure visibility throughout the procedure and offers valve-less access to the surgical site, which allows for intact specimen removal.

The gas sealed access cap of the subject disclosure includes a housing having a lid defining a central access port communicating with an interior cavity that receives pressurized gas through an inlet port to generate a gaseous sealing zone within the robotic cannula and that directs spent gas from the gaseous sealing zone through an outlet port. The inlet port and the outlet port of the housing communicate with a manifold associated with a bullseye connector fitting for communicating with a pressurized gas line and a return gas line of a filtered tube set.

A central access tube is aligned with the central access port of the housing and it extends distally from the interior cavity for communicating with a tubular portion of the robotic cannula. A distal end of the housing extends distally beyond a distal end of the central access tube for reception within a proximal bowl portion of the robotic cannula, and a pair of diametrically opposed flexible clips extend from a circumferential flange that is integrally formed with an exterior surface of the housing for releasably securing the access cap to the proximal bowl portion of the robotic cannula.

An annular jet assembly is supported in the interior cavity of the housing for generating the gaseous sealing zone within the robotic cannula so as to maintain a stable pressure within the surgical cavity of a patient. The annular jet assembly includes a central aperture aligned with the central access port of the lid. A plurality of circumferentially spaced apart radially inwardly extending vanes are formed integral with the housing and located within the interior cavity below the annular jet assembly for directing the spent gas from the gaseous sealing zone to the outlet port of the housing.

A circumferential groove is formed in the exterior surface of the housing distal to the diametrically opposed flexible clips for accommodating an O-ring seal. The circumferential groove is located proximal to the plurality of spaced apart vanes. The diametrically opposed flexible clips each include a proximal portion, a medial portion and a distal portion. The proximal portions of the diametrically opposed flexible clips are parallel to one another, the distal portions of the diametrically opposed flexible clips extend to the circumferential groove, and the circumferential flange is proximal to the circumferential groove.

The gas sealed access cap further includes an obturator having a proximal handle portion for cooperatively engaging with the lid of the housing and an elongated obturator shaft extending distally from the handle and having a distal cutting tip. An annular seal is supported on the obturator shaft at a location along the length thereof for sealing against an interior surface of the central access tube when the obturator shaft is extended through the central access port of the housing.

The subject disclosure is also directed to a new and useful method of performing a robotically assisted laparoscopic surgical procedure in an abdominal cavity of a patient. The method includes the steps of inserting a first robotic cannula into the abdominal cavity of the patient with a valve sealed cannula cap connected thereto, connecting a single lumen tube portion of a tube set to the valve sealed cannula cap, inserting a second robotic cannula into the abdominal cavity of the patient with a gas sealed cannula cap connected thereto, and connecting a dual lumen tube portion of the filtered tube set to the gas sealed cannula cap.

More particularly, the method includes providing a surgical access system including: a gas delivery device, a Veress needle, a valve sealed cannula cap, a gas sealed cannula cap having an obturator connected thereto, a first robotic cannula, a second robotic cannula and a filtered tube set that includes a filter cartridge, a single lumen tube portion and a dual lumen tube portion, wherein a removable plug is initially attached to a fitting at the distal end of the dual lumen tube portion of the filtered tube set.

The method further includes the steps of inserting the filter cartridge of the filtered tube set into a reception port of the gas delivery device, locking the filter cartridge within the reception port by rotating a mechanical lever arm of the gas delivery device and then confirming that a flow setting and a pressure setting on the gas delivery device are appropriate for the patient.

The method further includes the steps of inserting the Veress needle into the abdominal cavity of the patient, connecting the single lumen tube portion of the filtered tube set to a connector of the Veress needle, and then insufflating the abdominal cavity though the Veress needle until a set abdominal pressure is reached.

The method further includes the steps of connecting the gas sealed cannula cap to the second robotic cannula together with the obturator, inserting the first robotic cannula into the abdominal cavity of the patient with the valve sealed cannula cap connected thereto, and then inserting the second robotic cannula into the abdominal cavity of the patient with the gas sealed cannula cap connected thereto.

The method further includes the steps of removing the plug from the fitting at the distal end of the dual lumen tube portion of the filtered tube set, and then connecting the fitting of the dual lumen tube portion of the filtered tube set to a connector of the gas sealed cannula cap. The method further includes the steps of disconnecting the single lumen tube portion of the tube set from the Veress needle and then connecting the single lumen tube portion of the tube set to a connector of the valve sealed cannula cap, whereupon the gas delivery device will begin circulating pressurized gas through the gas sealed cannula by way of the dual lumen portion of the tube set and the gas delivery device will run a calibration process. Thereafter, the obturator is removed from the gas sealed cannula cap.

Upon the conclusion of the robotically assisted laparoscopic surgical procedure, the method involves the steps of replacing the obturator in the gas sealed cannula cap. At such a time, the circulation of pressurized gas by the gas delivery device is stopped and the method includes the step of disconnecting the dual lumen portion of the tube set from the connector of the gas sealed cannula cap.

These and other features of the devices, systems and methods of the subject disclosure will become more readily apparent from the following detailed description of the disclosed embodiments taken in conjunction with the drawings.

### BRIEF DESCRIPTION OF THE DRAWINGS

So that those skilled in the art will readily understand how to make and use the gas sealed access cap of the subject disclosure without undue experimentation, embodiments thereof will be described in detail herein below with reference to the figures wherein:
Fig. 1 is an illustration of an operating room containing a robotic surgical system with which the gas sealed access cap of the subject disclosure is employed;
Fig. 2 is a perspective view of a filtered tube set operatively associated with the gas sealed access cap of the subject disclosure as well as a valve sealed access cap used in conjunction therewith;
Fig. 3 is an illustration of the filtered tube set shown in Fig. 2 installed within a surgical gas delivery device, wherein the gas sealed access cap of the subject disclosure and two valve sealed access caps are inserted into a patients abdominal cavity, supported by respective robotic manipulator arms depicted in Fig. 1;
Fig. 4 is a perspective view of the gas sealed access cap of the subject disclosure separated from the proximal bowl portion of a robotic cannula;
Fig. 5 is a perspective view of an obturator separated from the gas sealed access cap, which is shown installed within the proximal bowl portion of a robotic cannula;
Fig. 6 is a perspective view of the obturator installed within the gas sealed access cap, which is installed within the proximal bowl portion of a robotic cannula;
Fig. 7 is an exploded perspective view of the gas sealed access cap of the subject disclosure with parts separated for ease of illustration;
Fig. 8 is an enlarged side elevational view of the connector manifold of the gas sealed access cap of the subject disclosure;
Fig. 9 is a cross-sectional view of the housing of the gas sealed access cap taken alone line 9-9 of Fig. 7;
Fig. 10 is a partial cross-sectional view taken alone line 10-10 of Fig. 6;
Fig. 11 is an illustration of the filtered tube set being inserted into a surgical gas delivery device;
Fig. 12 is an illustration of the filter cartridge of the filtered tube being locked into place by rotating a mechanical lever;
Figs. 13 and 14 illustrate the graphical user interface screen of the surgical gas delivery device providing a user with the ability to Confirm proper settings for an operating mode, gas flow and abdominal pressure;
Fig. 15 illustrates the connection of the gas sealed access cap in the proximal bowl portion of a robotic cannula, where an audible click is generated to ensure a secure connection;
Fig. 16 illustrates the insertion of a conventional Veress needle into the patient's abdominal cavity;
Fig. 17 illustrates the connection of insufflation tubing to the stopcock valve of the Veress needle shown in Fig. 16;
Fig. 18 illustrates the graphical user interface screen of the surgical gas delivery device providing a user with the ability to Start insufflation of the abdominal cavity;
Fig. 19 illustrates the graphical user interface screen of the surgical gas delivery device informing the user that the set abdominal Pressure has been reached, whereupon cannulas may be inserted into the abdominal cavity and the tube set can be connected to the cannulas with obturators in place;
Fig. 20 illustrates the insertion of the robotic cannula into the patient's abdominal cavity with the gas sealed access cap of the subject disclosure securely attached thereto;
Fig. 21 illustrates removal of the plug from the dual lumen fitting of the tube set so that the dual lumen portion of the tube set can be attached to the bulls-eye connector of the gas sealed access cap;
Fig. 22 illustrates connection of the insufflation tubing of the filtered tube set to the luer fitting of a valve sealed access cap attached to a robotic cannula;
Fig. 23 illustrates the graphical user interface screen of the surgical gas delivery device showing a user that the gas delivery device is automatically launching into a gas sealed usage mode, whereupon the system will calibrate with the obturators in place;
Fig. 24 illustrates the graphical user interface screen of the surgical gas delivery device once calibration is complete, whereupon the gas sealed usage mode is declared Active and the obturator can be removed from the gas sealed access cap;
Fig. 25 illustrates removal of the obturator from the gas sealed access cap after calibration is complete;
Fig. 26 illustrates the graphical user interface screen of the surgical gas delivery device providing a user with the ability to Stop the gas sealed usage mode, whereupon the obturators can be replaced before disconnecting the access caps from the filtered tube set to prevent loss of abdominal pressure; and
Fig. 27 illustrates a message on the graphical user interface screen of the surgical gas delivery device indicting that the surgical gas delivery device will undergo final calibration, after which the gas delivery device can be powered down and the filtered tube set can be removed from the gas delivery device and the cannulas.

### DETAILED DESCRIPTION OF THE EMBODIMENTS

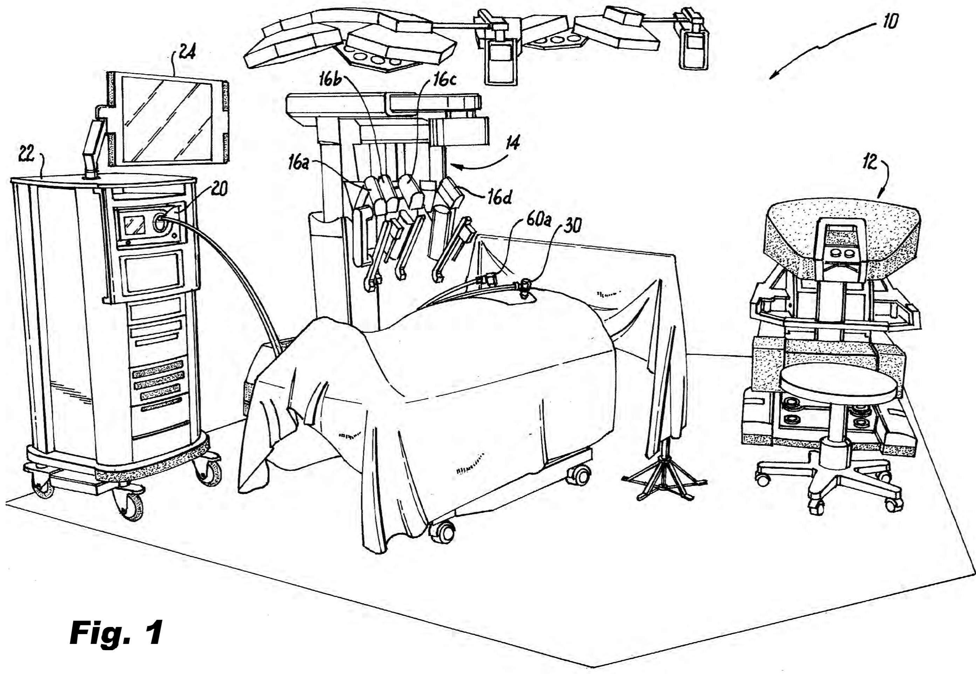

Referring now to the drawings, there is illustrated in Fig. 1 a surgical operating room containing a robotic surgical system 10 and a multi-modal gas delivery device 20 with which the novel gas sealed access cap 30 of the subject disclosure is employed to perform a laparoscopic surgical procedure on a patient.

The robotic surgical system 10 illustrated in Fig. 1 is depicted as the Da Vinci robotic surgical system, which is manufactured and sold by Intuitive Surgical, Inc. of Sunnyvale, CA. The system basically includes a console 12 where a surgeon would be seated to remotely perform a surgical procedure on the patient and a patient-side cart 14 with four interactive robotic arms 16a-16d that are controlled by the surgeon from the console 12.

The multi-modal gas delivery device 20 shown in Fig. 1 is depicted as an iFS Intelligent Flow System, which is manufactured and sold by Conmed Corporation, of Largo, FL. An example of such a device is disclosed in U.S. Patent No. 9,526,849, the disclosure of which is incorporated herein by reference in its entirety. The gas delivery device 20 is supported in a mobile tower cart 22 that has a monitor screen 24 to enable operating room staff to see what the surgeon is viewing at the console 12 intra-abdominally by way of the endoscope during the surgical procedure. Gas delivery device 20 is adapted and configured to operate in multiple modes to insufflate the patient's abdominal cavity and measure abdominal pressure, maintain a stable pneumoperitoneum and facilitate valve-less instrument access to the abdominal cavity, and facilitate smoke evacuation from the abdominal cavity during the surgical procedure.

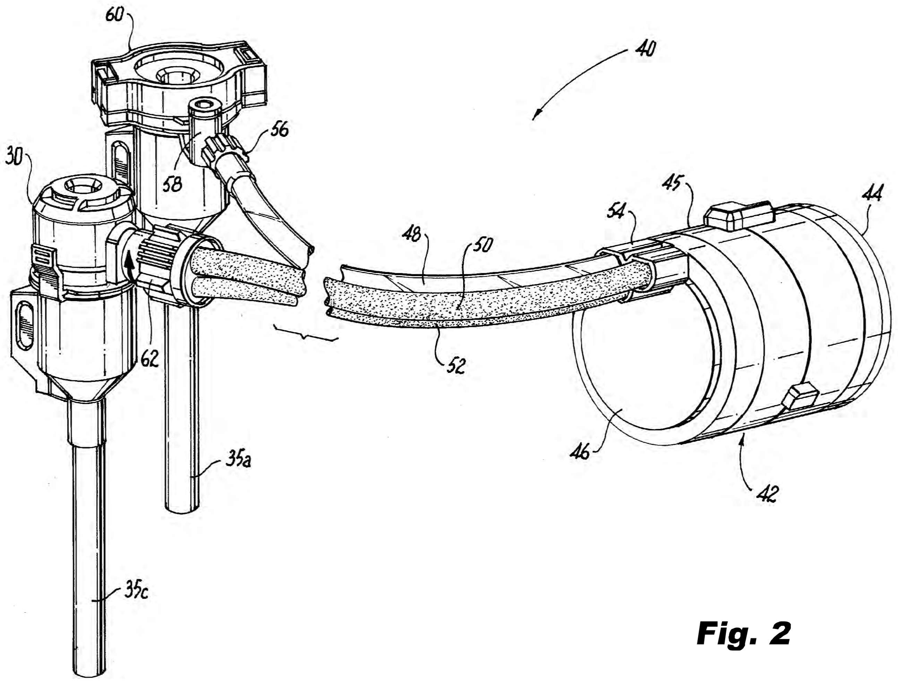

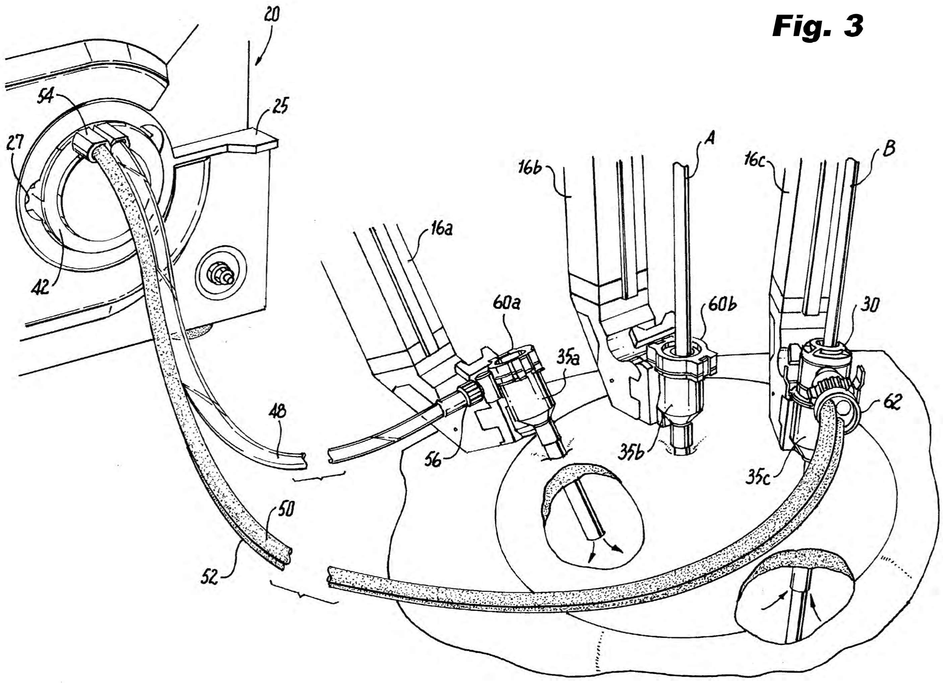

Turning now to Figs. 2 and 3, the gas sealed access cap 30 of the subject disclosure is shown in conjunction with a filtered tube set 40 and a valve sealed access cap 60. The gas sealed access cap 30 and the valve sealed access cap 60 are each operatively connected to a respective robotic cannula 35c and 35a, respectively. These are Da Vinci Xi/X type robotic cannulas. An example is disclosed in U.S. Patent No. 10,463,395, which is assigned to Intuitive Surgical Operation Inc., and incorporated herein by reference in its entirety. The valve sealed access cap 60 is also manufactured by Intuitive Surgical, Inc. Similar examples of valve sealed access caps are disclosed in U.S. Patent No. 10,463,395.

The filtered tube set 40 shown in Fig. 2 includes a disposable filter cartridge 42 dimensioned and configured for insertion into a reception port 27 of the gas delivery device 20. A rotatable lever arm 25 is used to lock the filter cartridge 42 in the reception port 27. The filter cartridge 42 includes a generally cylindrical housing 45 that defines a plurality of internal filtered flow passages (not shown), which extend between a rear end cap 44 and a front end cap 46. A filter cartridge of this type is disclosed in commonly assigned U.S. Patent No. 9,067,030, the disclosure of which is incorporated herein by reference in its entirety. The internal filtered flow passages of the filter cartridge are illustrated and described therein.

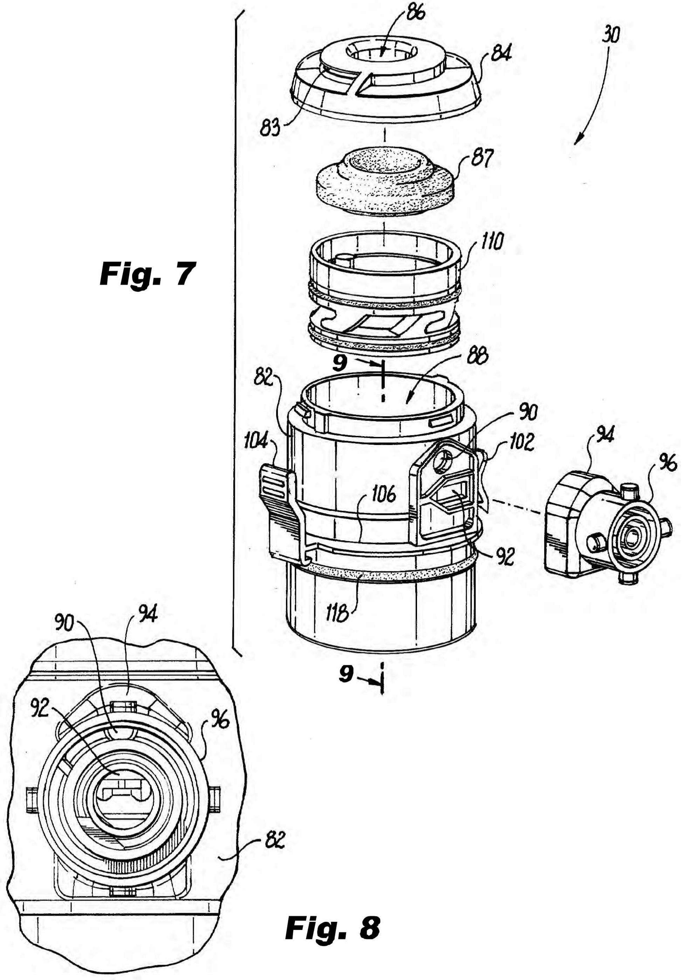

An insufflation and sensing lumen or tube 48, a gas supply lumen or tube 50 and a gas return lumen or tube 52 extend from a manifold 54 on the front end cap 46 of the filter cartridge 42. In an embodiment of the disclosure, the insufflation lumen 48 consist of clear tubing, while the gas supply tube 50 and gas return tube 52 consist of colored tubing. This helps to visually differentiate these tubes from one another in the operating room. The insufflation and sensing lumen 48 has a standard luer fitting 56 at the distal end thereof for mating with a luer connector 58 of the valve sealed access cap 60. The gas supply lumen 50 and the gas return lumen 52 extend distally to a common multi-lumen bulls-eye fitting 62 for mating with a multi-lumen bulls-eye connector 96 on the gas sealed access cap 30 (see Fig. 8).

The bull-eye fitting 62 is of the tri-lumen type disclosed in commonly assigned U.S. Patent No. 9,526,886, the disclosure of which is incorporated herein by reference in its entirety. The bull-eye fitting 62 differs somewhat from the tri-lumen fitting disclosed in U.S. Patent No. 9,526,886 in that only two of the three gas flow passages of the fitting are utilized for gas flow. More particularly, the gas delivery and gas return passages of fitting 62 are utilized. The remaining unused flow passage is purposefully plugged during manufacture, as best seen in Fig. 3. The gas delivery device 20 is programmed to recognize this difference.

It is envisioned however, and well within the scope of the subject disclosure, that the fitting 62 could be constructed as a dual-lumen bulls-eye fitting with only two gas flow passages, as disclosed for example in commonly assigned U.S. Patent No. 10,736,657, which is incorporated herein by reference in its entirety. Other types of dual-lumen fittings and connectors could also be employed, such as those disclosed in commonly assigned U.S. Patent No. 11,065,430, which is also incorporated herein by reference in its entirety.

As illustrated in Fig. 3, the robotic arms 16a-16c of the patient-side cart 14 are used to grasp and manipulate respective robotic cannulas 35a-35c during a robotically assisted laparoscopic surgical procedure. More particularly, a first robotic arm 16a is shown grasping a first robotic cannula 35a that is associated with a valve sealed access cap 60a connected to the insufflation and sensing lumen 48 of tube set 40 by way of luer fitting 56. A second robotic arm 16b is shown grasping a second robotic cannula 35b that is associated with a second valve sealed access cap 60b, and a third robotic arm 16c is shown grasping a third robotic cannula 35c associated with the gas sealed access cap 30 of the subject disclosure. The gas sealed access port 30 is connected to the gas supply lumen 50 and the gas return lumen 52 by way of the bulls-eye fitting 62.

In this exemplary illustration of Fig. 3, the first valve sealed access cap 60a connected to lumen 48 is used for insufflation of the patient's abdominal cavity and to enable the gas delivery device 20 to periodically sense abdominal pressure. This valve first sealed access cap can also be used for instrument access. The second valve sealed access port 60b is used to provide abdominal access for the laparoscope A, and the gas sealed access port 30 connected to lumens 50 and 52 is used to maintain a stable pneumoperitoneum, provide valve-less access for surgical instrument B, and enable smoke evacuation from the abdominal cavity during the surgical procedure. Those skilled in the art will readily appreciate that the size, orientation and/or arrangement of the robotic cannulas and the surgical instruments shown in Fig. 3 could vary depending upon patient anatomy and the surgical procedure being performed.

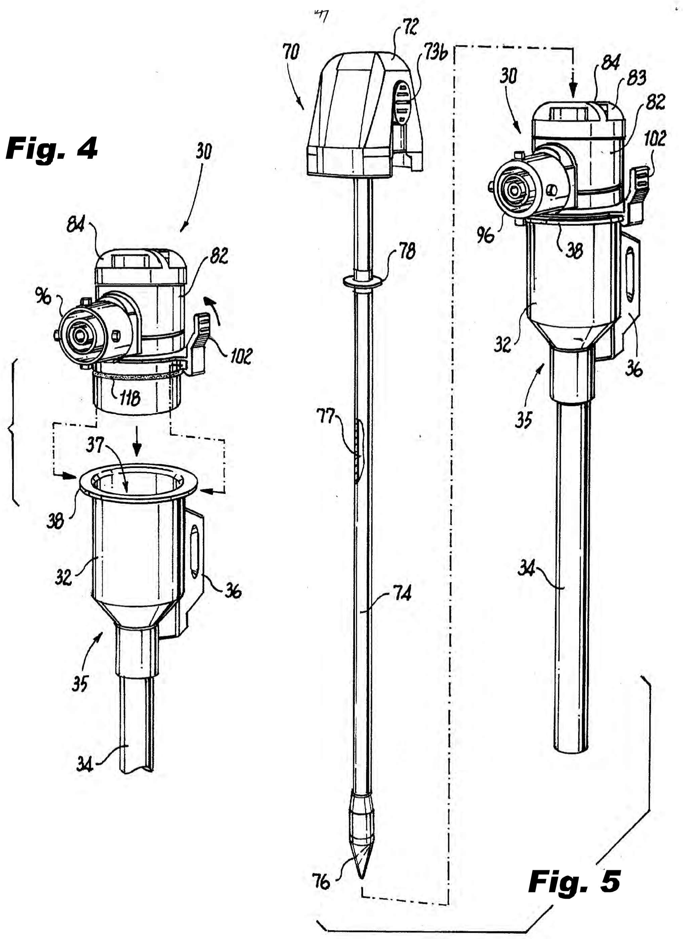

Referring now to Figs. 4 through 6, the gas sealed access cap 30 of the subject disclosure is shown in conjunction with a robotic cannula 35 and an obturator 70. The obturator 70 is adapted and configured to facilitate the percutaneous introduction of the robotic cannula 35 into the abdominal cavity of a patient during a surgical procedure. This is typically done by a surgeon under vision through a small incision in the abdominal wall.

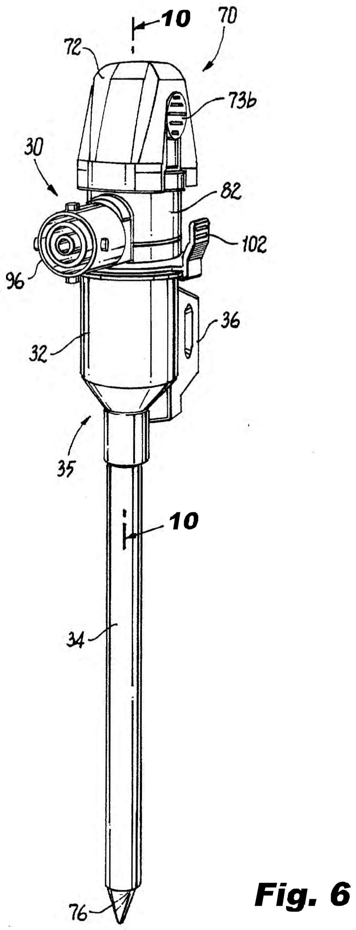

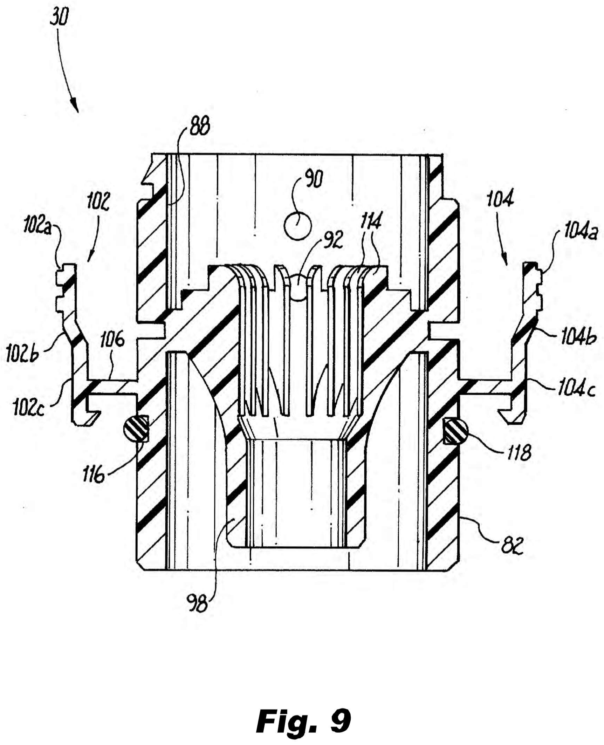

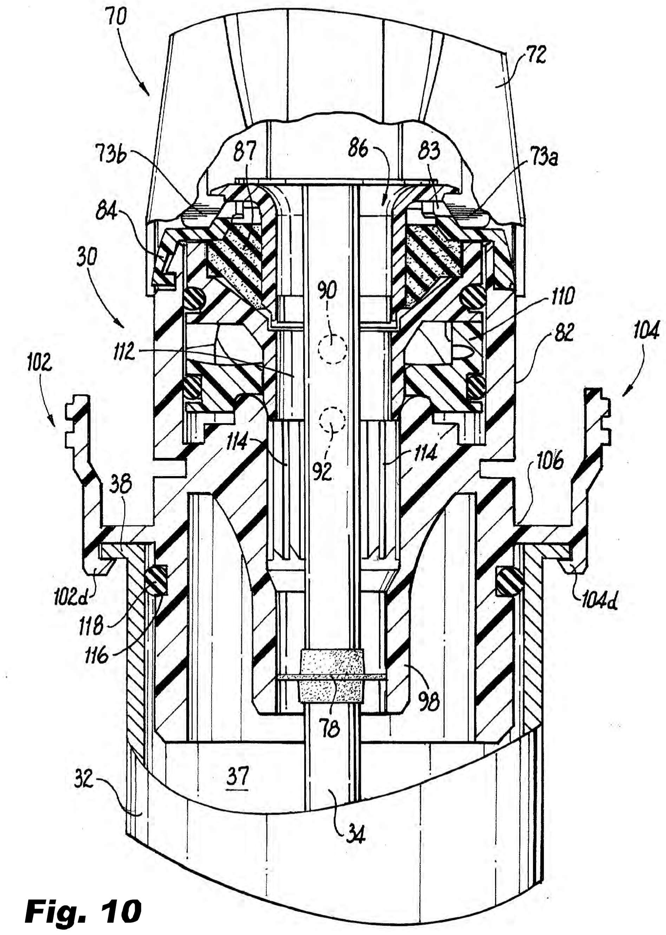

The robotic cannula 35 (i.e., the Da Vinci X/Xi robotic cannula) includes a generally cylindrical proximal bowl portion 32 and an elongated tubular body portion 34 that extends distally from the proximal bowl portion 32. The proximal bowl portion 32 includes an interior bowl area 37 that is dimensioned and configured to receive the gas sealed access cap 30. A grasping handle 36 extends radially outward from the exterior wall of the proximal bowl portion 32 to facilitate manipulation of the cannula 35 by a robotic arm 16a-16d, as shown in Fig. 3. An annular engagement flange 38 is formed at the top end of the proximal bowl portion 32 of cannula 35. The engagement flange 38 cooperates with a pair of diametrically opposed cantilevered flexible engagement clips 102 and 104 that are formed integral with the access cap 30, as shown in Figs. 5 and 6. The engagement clips 102, 104 and the way in which they cooperate and releasably engage with flange 38 will be described in greater detail below with reference to Figs. 9 and 10.

When the access cap 30 is inserted into the interior bowl area 37 of the proximal bowl portion 32 of robotic cannula 35, as depicted in Fig. 4, an annular O-ring seal 118 seated on an exterior surface of access cap 30 will sealingly engage the interior surface of bowl area 37. This sealing interface will also be described in greater detail below with reference to Figs. 9 and 10.

With continuing reference to Figs. 5 and 6, the obturator 70 is of the type disclosed in commonly assigned U.S. Patent No 9,545,264, the disclosure of which is incorporated herein by reference in its entirety. Obturator 70 has a proximal handle portion 72 with a pair of diametrically opposed spring-biased clasps 73a, 73b for cooperatively engaging with a pair of diametrically opposed recesses 83 in the lid 84 of the housing (see Fig. 10).

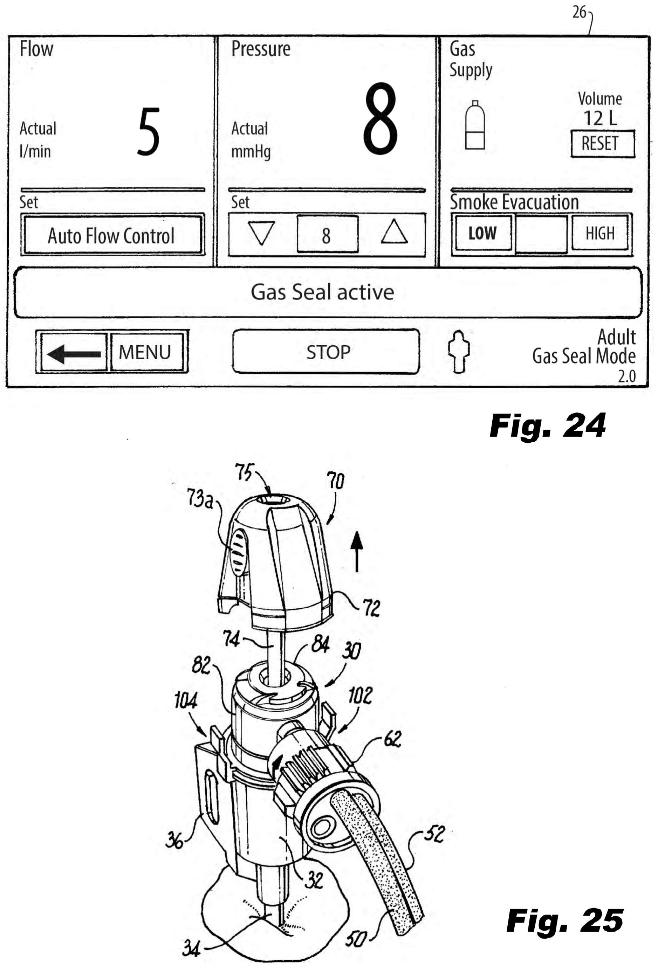

An elongated tubular obturator shaft 74 extends distally from the handle portion 72 of obturator 70. The shaft 74 has a transparent optical cutting tip 76 at the distal end thereof for visualization during insertion. An annular seal 78 is supported on the obturator shaft 74 at a location along the length thereof for interacting with an interior surface of the access cap 30, which will be described in further detail below with reference to Fig. 10. The proximal handle portion 72 of the obturator 70 defines a central port 75 (see Fig. 25). The central port 75 in handle portion 72 communicates with a central bore 77 that extends through the elongated obturator shaft 74 to the distal optical cutting tip 76 (see Fig. 5). The central bore 77 in shaft 74 is dimensioned and configured to accommodate a rigid scope for visualization through the cutting tip 76 during insertion through the patient's abdominal wall.

Referring now to Figs. 7 through 10, the gas sealed access cap 30 includes a generally cylindrical housing 82 formed in an injection molding process from a lightweight, medical grade thermoplastic material. The material can be transparent, translucent or opaque. The housing 82 of access cap 30 has an interior cavity 88 that is enclosed by a cover or lid 84, which defines a central access port 86. Diametrically opposed recesses 83 are formed in the lid 84 to cooperate with the diametrically opposed spring-biased clasps 73a and 73b on obturator housing 72, as shown previously in Fig. 6.

The interior cavity 88 of housing 82 receives pressurized gas from a pump in the gas delivery device 20 through an inlet port 90. The pressurized gas is used to generate a gaseous sealing zone within the robotic cannula 35. The gaseous sealing zone maintains a stable pneumoperitoneum and facilitates valve-less, gas sealed instrument access to the abdominal cavity through cannula 35. Spent gas used to create the gaseous sealing zone within the robotic cannula 35 is directed from the interior cavity 88 of housing 82 through an outlet port 92 and back to the gas delivery device 20 by way of filter cartridge 42 for recirculation.

Referring to Figs. 7 and 8, the inlet port 90 and the outlet port 92 of the housing 82 communicate with a manifold 94 associated with a multi-lumen bulls-eye connector 96, which is designed to communicate with the bulls-eye fitting 62 of tube set 40. The bulls-eye connector 96 and the way in which it cooperates with the fitting 62 is described in more detail in commonly assigned U.S. Patent No. 9,526,886.

As best seen in Fig. 7, a two-piece annular jet assembly 110 is sealingly supported in the interior cavity 88 of the housing 82 of access cap 30 for generating the gaseous sealing zone within the robotic cannula 35 so as to maintain a stable pressure within the surgical cavity of a patient. The annular jet assembly 110 is of the type disclosed in commonly assigned U.S. Patent No. 9,907,569, the disclosure of which is incorporated herein by reference in its entirety. The jet assembly 110 includes a central aperture 112 aligned with the central access port 86 of the lid 84 (see Fig. 10). A sound attenuating disc 87 made from a foam material is positioned within the housing 82 between the lid 84 and the annular jet assembly 110. The foam disc 87 functions to filter, attenuate or otherwise reduce noise that may result from the jet assembly 110 generating the gaseous sealing zone.

Referring now to Figs. 9 and 10, a plurality of circumferentially spaced apart radially inwardly extending vanes or fins 114 are formed integral with the housing 82 and located within the interior cavity 88 thereof below the annular jet assembly 110. These vanes 114 are adapted and configured to direct the spent gas from the gaseous sealing zone in the cannula 35 to the outlet port 92 of the housing 82. The arrangement of vanes or fins 114 is similar to the arrangement of vanes disclosed in commonly assigned U.S. Patent No. 8,795,223, the disclosure of which is incorporated herein by reference in its entirety. In that patent however, the circumferentially spaced apart vanes are formed in a separate nested insert, rather than being formed integral with the housing 82 of the access cap 30.

A central access tube 98 extends distally from the arrangement of circumferential vanes 114 within the lower portion of the interior cavity 88, aligned with the central access port 86 in the lid 84 of the housing 82 and the central aperture 112 of jet assembly 110. The central access tube 98 communicates with the interior bowl area 37 and the central bore of the tubular body portion 34 of the robotic cannula 35, as best seen in Fig. 10. A distal end of the housing 82 of access cap 30 extends distally beyond a distal end of the central access tube 98, as best seen on Fig. 9.

The dimensional relationship between the distal end of the housing 82 and the distal end of the central access tube 98 ensures that the gas sealed access cap 30 will be properly seated within the proximal bowl portion 32 of the robotic cannula 35 when the two components are connected together. As described above, an annular seal 78 is supported on the obturator shaft 74 of obturator 70 at a location along the length thereof. More particularly, the annular seal 78 is located to seal against an interior surface central access tube 98 adjacent the distal end thereof, as shown in Fig. 10. This prevents the egress of insufflation gas through the gas sealed access cap 30 at certain times during the surgical procedure, such as during calibration or when the gaseous sealing mode is not active.

As mentioned above, a pair of diametrically opposed cantilevered flexible engagement clips 102 and 104 are formed integral with the housing 82 of access cap 30 for releasably engaging the annular flange 38 at the top of the proximal bowl portion 32 of robotic cannula 35. More particularly, the engagement clips 102 and 104 are formed integral with and operatively connected to a circumferential flange 106 that extends radially outward from the exterior surface of the housing 82 of access cap 30.

The two engagement clips 102, 104 each include a proximal portion 102a, 104a, a medial portion 102b, 104b and a distal portion 102c, 104c. The proximal portions 102a, 104a of the flexible clips 102, 104 extend parallel to one another and have outer gripping surfaces that can be pressed or flexed inwardly by a user to release or otherwise dis-engage the clips 102, 104 from the flange 38. The medial portions 102b, 104b are angled inwardly toward the housing 82 and provide a transition to the distal portions 102c, 104c. The distal portions 102c, 104c of clips 102, 104 are integrally formed with respective radially outwardly extending opposed lateral bridges of the integral circumferential flange 106. The distal ends of the distal portions 102c, 104c of clips 102, 104 are turned radially inwardly to form diametrically opposed feet 102d, 104d that releasably engage below the annular flange 38 in a mechanically retaining manner, which is best seen in Fig. 10.

As described previously, when the access cap 30 is inserted into the interior bowl area 37 of the proximal bowl portion 32 of robotic cannula 35, an annular O-ring seal 118 seated on an exterior surface of access cap 30 will sealingly engage the interior surface of bowl area 37. The O-ring seal 118 is seated in a circumferential groove 116 formed in the exterior surface of the housing 82, as best seen in Fig 9. The O-ring seal 118 is located distal to the opposed feet 102d, 104d of the flexible clips 102, 104. This ensures that the O-ring seal 118 is properly seated against the interior wall of the interior bowl area 37 of the proximal bowl portion 32 of robotic cannula 35, which is best seen in Fig. 10.

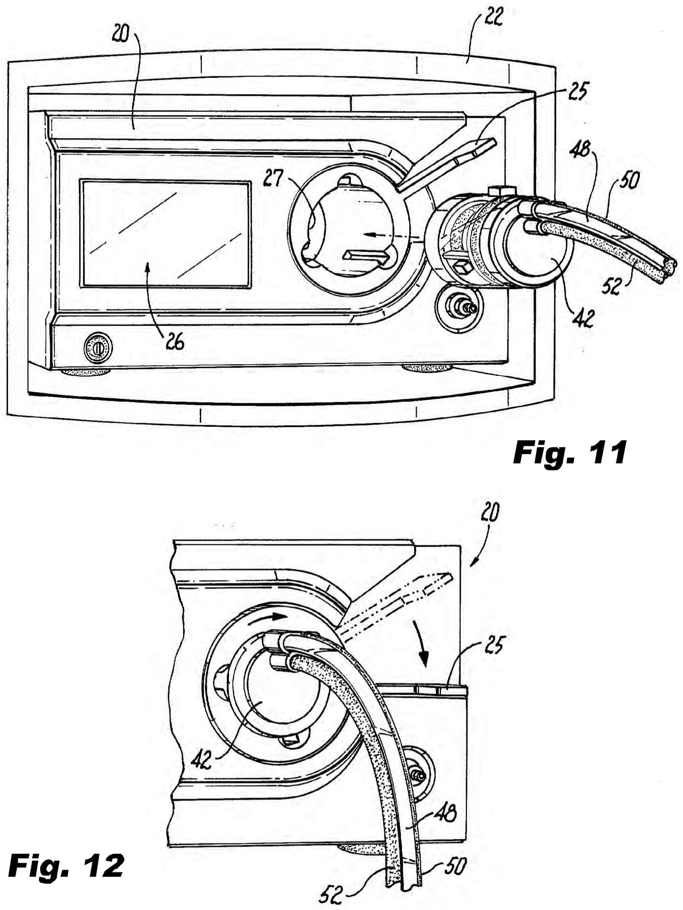

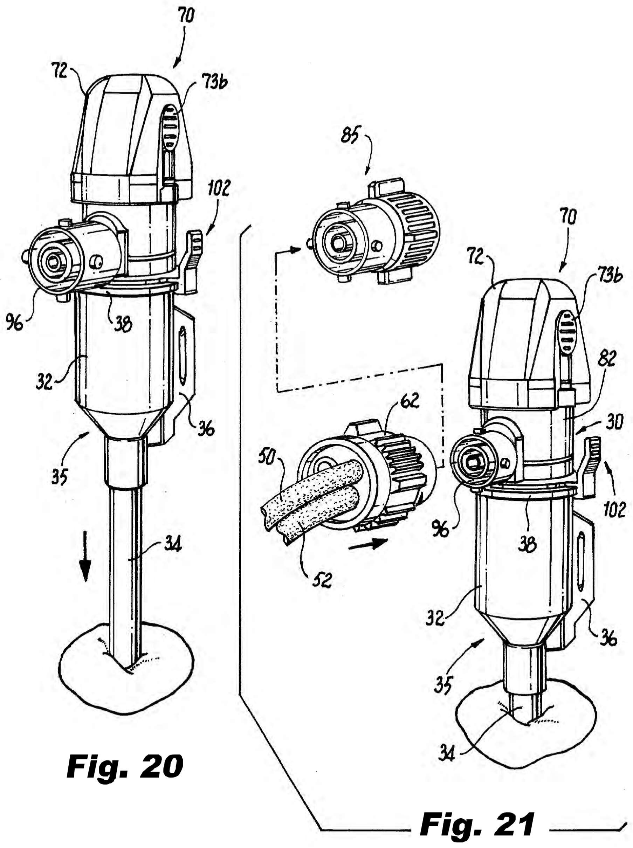

Referring now to Figs. 11 through 27, to perform a robotically assisted laparoscopic surgical procedure in accordance with the subject disclosure, the method includes providing a surgical access system that includes: a gas delivery device 20, a standard Veress needle 120, a gas sealed access cap 30, a valve sealed access cap 60 having an obturator 70 connected thereto, a first robotic cannula 35a, a second robotic cannula 35b and a filtered tube set 40 that includes a filter cartridge 42, a single lumen portion including an insufflation/sensing lumen 48 and a dual lumen portion including a gas supply lumen 50 and a gas return lumen 52. A removable plug 85 is attached to the fitting 62 at the distal end of the dual lumen portion 50, 52, as best seen in Fig. 21.

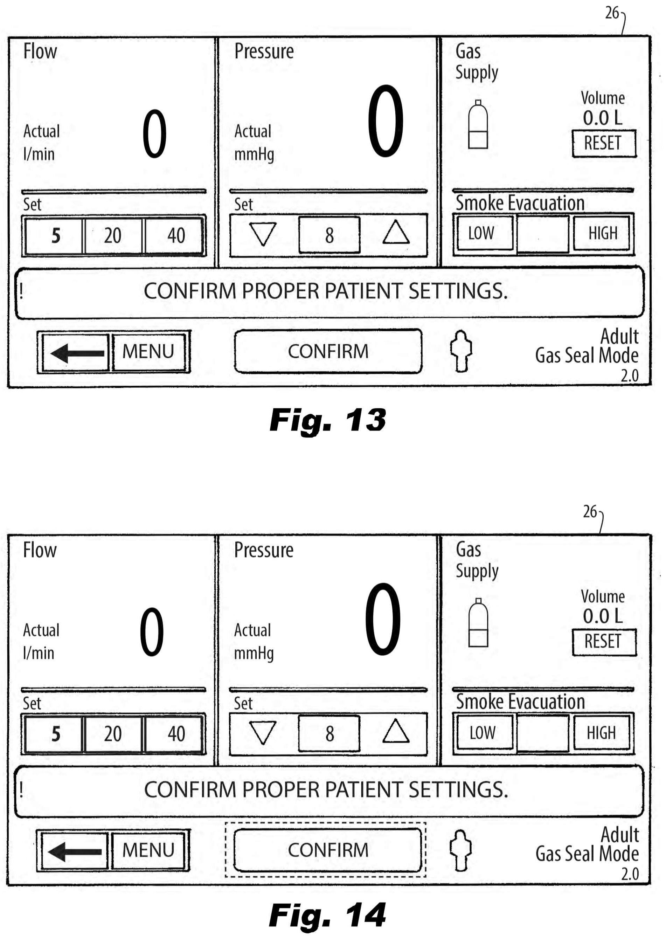

The method includes initially inserting the filter cartridge 42 of the filtered tube set 40 into the reception port 27 of the gas delivery device 20, as shown in Fig. 11, and then locking the filter cartridge 42 within the reception port 27 by rotating the mechanical lever arm 25 of the gas delivery device 20, as shown in Fig. 12. At such a time, the user refers to the graphical user interface (GUI) screen 26 of the gas delivery device 20 to confirm that a flow setting in l/min and a pressure setting in mmHg on the gas delivery device 20 are appropriate for the patient, as depicted in Fig. 13. The gas delivery device 20 can operate in a Pediatric Mode or an Adult Mode, which have different flow and pressure settings. If the settings are correct, the user will press the CONFIRM button, as shown in Fig. 14.

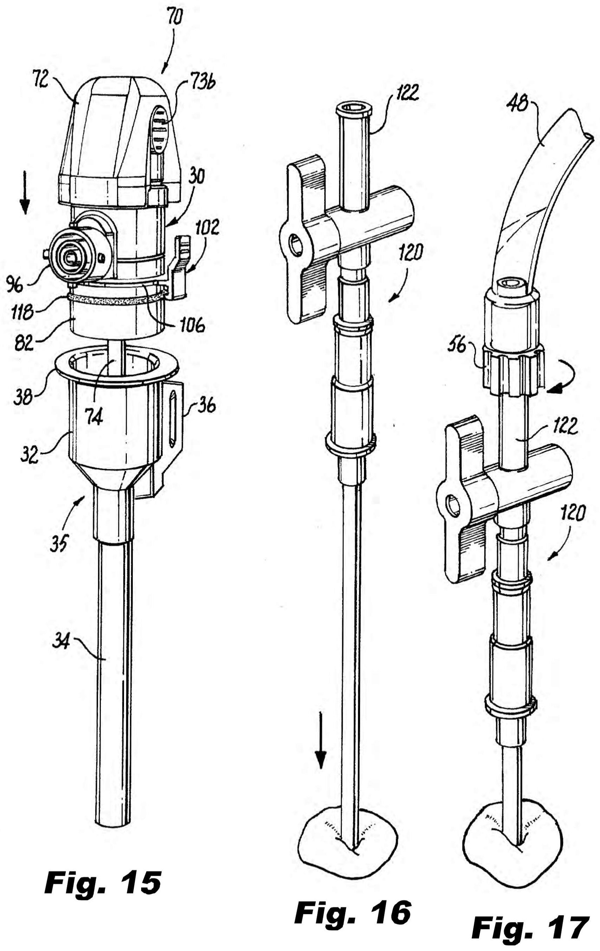

The method further includes the step of connecting the gas sealed access cap 30 to the robotic cannula 35 together with the obturator 70, as shown in Fig. 15. An audible click is generated to ensure a secure connection between the cannula cap 30 and the cannula 35. This audible click will be generated by the mechanical interaction of the flexible engagement clips 102, 104 and the annular flange 38 of the cannula 35.

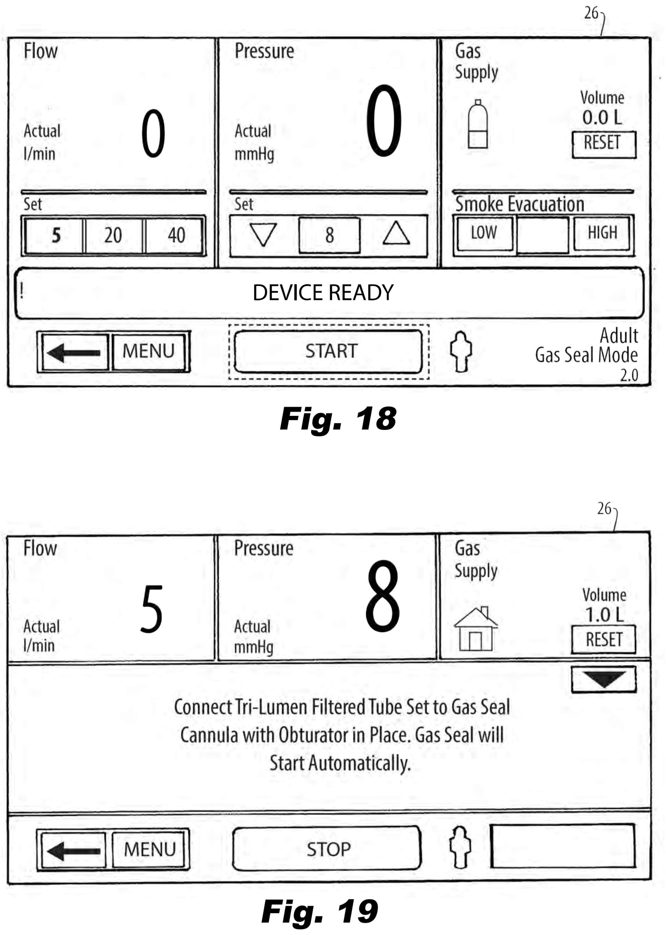

The method further includes the steps of percutaneously inserting the Veress needle 120 into the abdominal cavity of the patient, as shown in Fig. 16, and then connecting the fitting 56 single lumen tube portion 48 of the filtered tube set 40 to a luer connector 122 of the Veress needle 120, as shown in Fig. 17. Thereafter, the GUI screen 26 of gas delivery device 20 indicates Device Ready, and the user presses the Start button to begin the initial insufflation of the abdominal cavity though the Veress needle 120, as shown in Fig, 18. When a set abdominal pressure is reached (e.g., 8 mmHg), a message will be displayed on the GUI screen 26, indicating that the user can safely connect the dual lumen portion 50, 52 of the tube set 40 to the gas sealed cannula cap 30, as illustrated in Fig. 19. At such a time, the plug 85 on the bulls-eye fitting 62 at the distal end of the dual lumen portion 50, 52 can be removed.

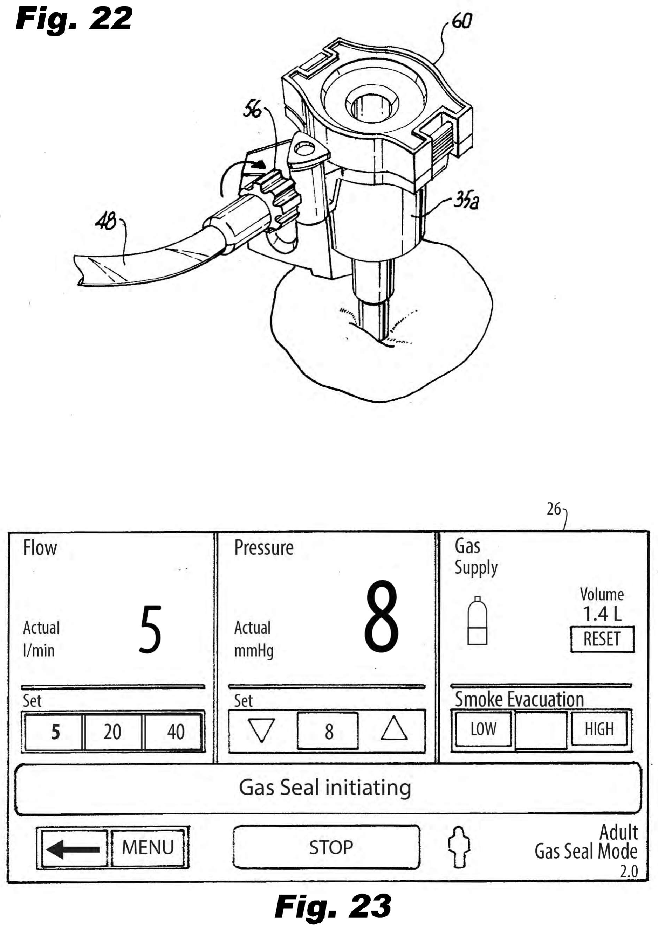

The method further includes the step of inserting robotic cannula 35a into the abdominal cavity of the patient with the valve sealed cannula cap 60 connected thereto (see Fig. 22), an then inserting robotic cannula 35 into the abdominal cavity of the patient with the gas sealed cannula cap 30 using the obturator 70 connected thereto, as shown in Fig. 20. Then, the bulls-eye fitting 62 associated with the dual lumen portion 50, 52 of the filtered tube 40 set is inserted onto and rotatably engaged with the connector 96 of the gas sealed cannula cap 30, as shown in Fig. 21. Those skilled in the art will readily appreciate that the gas sealed cannula should be inserted last, after all valve sealed cannulas involved in the procedure have been placed. This allows for the firmest abdominal wall during insertion of the cannula with the gas sealed cannula cap 30.

Once the cannula 35a with the valve sealed cannula cap 60 has been placed, the fitting 56 at the distal end of the single lumen tube portion 48 of the tube set 40 is disconnected from the Veress needle 120, and it is then rotatably connected to the luer connector of the valve sealed cannula cap 60, as shown in Fig. 22. Thereupon, the gas delivery device 20 will automatically begin circulating pressurized gas through the gas sealed cannula 30 by way of the dual lumen portion 50, 52 of the tube set 40 and the gas delivery device 20 will run a calibration process.

Fig. 23 illustrates the graphical user interface screen 26 of the surgical gas delivery device 20 showing a user that the gas delivery device 20 is automatically initiating or otherwise launching into a gas sealed usage mode, whereupon the system will calibrate with the obturators (e.g., obturator 70) in place. At this time, the seal 78 on obturator shaft 74 prevent the egress of insufflation gas through access cap 30. The graphical user interface screen 26 of the surgical gas delivery device 20 will provide a message indicting that the gas sealed usage mode is Active, once calibration is complete, as depicted in Fig. 24. Then the user removes the obturator 70 from the gas sealed cannula cap 30, as shown in Fig. 25, and the robotically assisted laparoscopic surgical procedure can commence.

Upon the conclusion of the robotically assisted laparoscopic surgical procedure, the method involves the steps of replacing the obturator 70 in the gas sealed cannula cap 30. At such a time, the circulation of pressurized gas by the gas delivery device 20 is stopped, and the fitting 62 at the distal end of the dual lumen portion 50, 52 of the tube set 40 is disconnected from the connector 96 of the gas sealed cannula cap 30.

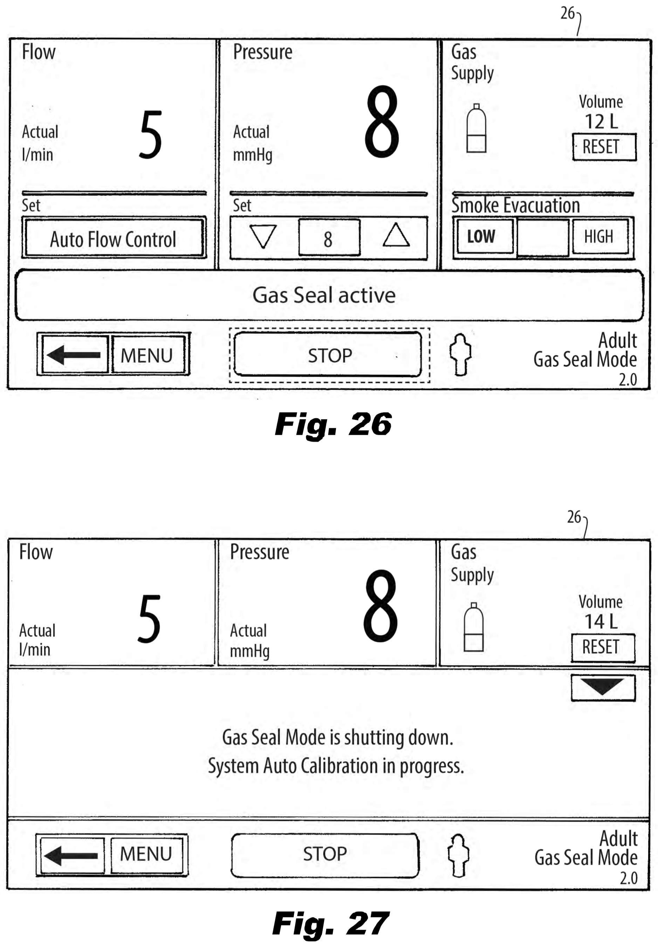

Fig. 26 illustrates the graphical user interface screen 26 of the surgical gas delivery device 20 providing a user with the ability to Stop the gas sealed usage mode, whereupon the obturators 70 can be replaced in the access caps 30, 60 before disconnecting the cannulas 35 from the filtered tube set 40 to prevent loss of abdominal pressure. The GUI shown in Fig. 27 illustrates that the system will undergo a final calibration, after which the gas delivery device 20 can be powered down and the filtered tube set 40 can be unlatched and removed from the gas delivery device 20. Thereupon, the robotically assisted laparoscopic procedure will conclude.

While the subject disclosure has been shown and described with reference to preferred embodiments, those skilled in the art will readily appreciate that changes and/or modifications may be made thereto without departing from the spirit or scope of the subject disclosure.

### The present invention can also be described by means of the following clauses:

1. A gas sealed access cap for a robotic cannula comprising:
   a) a housing having a lid defining a central access port communicating with an interior cavity that receives pressurized gas through an inlet port to generate a gaseous sealing zone within the robotic cannula and that directs spent gas from the gaseous sealing zone through an outlet port;
   b) a central access tube aligned with the central access port of the housing and extending distally from the interior cavity for communicating with a tubular portion of the robotic cannula, wherein a distal end of the housing extends distally beyond a distal end of the central access tube for reception within a proximal bowl portion of the robotic cannula; and
   c) a pair of diametrically opposed flexible clips extending from a circumferential flange that is integrally formed with an exterior surface of the housing for releasably securing the access cap to the proximal bowl portion of the robotic cannula.
2. A gas sealed access cap as recited in Clause 1, wherein an annular jet assembly is supported in the interior cavity of the housing for generating the gaseous sealing zone within the robotic cannula so as to maintain a stable pressure within the surgical cavity of a patient, and wherein the annular jet assembly includes a central aperture aligned with the central access port of the lid.
3. A gas sealed access cap as recited in Clause 2, wherein a plurality of circumferentially spaced apart radially inwardly extending vanes are formed integral with the housing and located within the interior cavity below the annular jet assembly for directing the spent gas from the gaseous sealing zone to the outlet port of the housing.
4. A gas sealed access cap as recited in Clause 1, wherein a circumferential groove is formed in the exterior surface of the housing distal to the diametrically opposed flexible clips for accommodating an O-ring seal.
5. A gas sealed access cap as recited in Clause 1, wherein the circumferential groove is located proximal to the plurality of spaced apart vanes.
6. A gas sealed access cap as recited in Clause 1, wherein the diametrically opposed flexible clips each include a proximal portion, a medial portion and a distal portion.
7. A gas sealed access cap as recited in Clause 6, wherein the proximal portions of the diametrically opposed flexible clips are parallel to one another.
8. A gas sealed access cap as recited in Clause 4, wherein the distal portions of the diametrically opposed flexible clips extend to the circumferential groove.
9. A gas sealed access cap as recited in Clause 4, wherein the circumferential flange is proximal to the circumferential groove.
10. A gas sealed access cap as recited in Clause 1, further comprising an obturator having a proximal handle portion for cooperatively engaging with the lid of the housing and an elongated obturator shaft extending distally from the handle and having a distal optical cutting tip.
11. A gas sealed access cap as recited in Clause 10, wherein the proximal handle portion of the obturator defines a central port communicating with a bore that extends through the elongated obturator shaft to the distal optical cutting tip for receiving a scope.
12. A gas sealed access cap as recited in Clause 10, wherein an annular seal is supported on the obturator shaft at a location along the length thereof for sealing against an interior surface of the central access tube when the obturator shaft is extended through the central access port of the housing.
13. A gas sealed access cap as recited in Clause 1, the inlet port and the outlet port of the housing communicate with a manifold associated with a bullseye connector fitting for communicating with a pressurized gas line and a return gas line of a filtered tube set.
14. A gas sealed access cap for a robotic cannula comprising:
   a) a housing having a lid defining a central access port extending to an interior cavity that supports an annular jet assembly for receiving pressurized gas from an inlet port of the housing, wherein the annular jet assembly includes a central aperture aligned with the central access port of the lid and is adapted to generate a gaseous sealing zone within the robotic cannula to maintain a stable pressure within the surgical cavity of a patient;
   b) a plurality of circumferentially spaced apart radially inwardly extending vanes formed integral with the housing and located within the interior cavity thereof below the annular jet assembly for directing spent gas from the gaseous sealing zone to an outlet port of the housing;
   c) a central access tube aligned with the central access port of the housing and the central aperture of the annular jet assembly, and extending distally from the interior cavity of the housing below the vanes for communicating with a tubular portion of the robotic cannula, wherein a distal end of the housing extends distally beyond a distal end of the central access tube for reception within a proximal bowl portion of the robotic cannula; and
   d) a pair of diametrically opposed flexible clips extending from a circumferential flange that is integrally formed with an exterior surface of the housing for releasably securing the access cap to the proximal bowl portion of the robotic cannula.
15. A gas sealed access cap as recited in Clause 14, wherein a circumferential groove is formed in the exterior surface of the housing distal to the opposed flexible clips for accommodating an O-ring seal.
16. A gas sealed access cap as recited in Clause 14, wherein the circumferential groove is located proximal to the spaced apart vanes.
17. A gas sealed access cap as recited in Clause 14, wherein the diametrically opposed flexible clips each include a proximal portion, a medial portion and a distal portion.
18. A gas sealed access cap as recited in Clause 17, wherein the proximal portions of the diametrically opposed flexible clips are parallel to one another.
19. A gas sealed access cap as recited in Clause 17, wherein the distal portions of the diametrically opposed flexible clips extend to the circumferential groove.
20. A gas sealed access cap as recited in Clause 15, wherein the circumferential flange is proximal to the circumferential groove.
21. A gas sealed access cap as recited in Clause 14, further comprising an obturator having a proximal handle portion for cooperatively engaging with the lid of the housing and an elongated obturator shaft extending distally from the handle and having a distal cutting tip.
22. A gas sealed access cap as recited in Clause 21, wherein the proximal handle portion of the obturator defines a central port communicating with a bore that extends through the elongated obturator shaft to the distal optical cutting tip for receiving a scope.
23. A gas sealed access cap as recited in Clause 21, wherein an annular sealing flange is supported on the obturator shaft at a location along the length thereof for sealing against an interior surface of the central access tube when the obturator shaft is extended through the central access port of the housing.
24. A gas sealed access cap as recited in Clause 14, the inlet port and the outlet port of the housing communicate with a manifold associated with a bullseye connector fitting for communicating with a pressurized gas line and a return gas line of a filtered tube set.
25. A method of performing a robotically assisted laparoscopic surgical procedure in an abdominal cavity of a patient, comprising the steps of:
   a) insufflating the abdominal cavity of a patient;
   b) connecting a gas sealed cannula cap to a robotic cannula;
   c) inserting the robotic cannula into the insufflated abdominal cavity; and
   d) connecting a tube set to the gas sealed cannula cap.
26. The method of Clause 25, wherein the step of insufflating the abdominal cavity of a patient includes:
   a) inserting a Veress needle into the abdominal cavity of the patient;
   b) connecting a single lumen tube portion of the tube set to the Veress needle; and
   c) insufflating the abdominal cavity though the Veress needle until a set abdominal pressure is reached.
27. The method of Clause 25, wherein the step of connecting a tube set to the gas sealed cannula cap includes the steps of removing a plug from a fitting on a distal end of a dual lumen tube portion of the tube set and connecting the fitting to the gas sealed cannula cap.
28. The method of Clause 25, further comprising the step of inserting a second robotic cannula into the abdominal cavity of the patient with a valve sealed cannula cap connected thereto.
29. The method of Clause 28, further comprising the steps of disconnecting the single lumen tube portion of the tube set from the Veress needle and subsequently connecting the single lumen tube portion of the tube set to the valve sealed cannula cap.
30. A method of performing a robotically assisted laparoscopic surgical procedure in an abdominal cavity of a patient, comprising the steps of:
   a) inserting a first robotic cannula into the abdominal cavity of the patient with a valve sealed cannula cap connected thereto;
   b) connecting a single lumen tube portion of a tube set to the valve sealed cannula cap; and
   c) inserting a second robotic cannula into the abdominal cavity of the patient with a gas sealed cannula cap connected thereto; and
   d) connecting a dual lumen tube portion of the filtered tube set to the gas sealed cannula cap.
31. The method of Clause 30, further comprising the step of insufflating the abdominal cavity of the patient though a Veress needle.
32. The method of Clause 30, further comprising the step connecting the single lumen tube portion of the tube set to the Veress needle.
33. A method of performing a robotically assisted laparoscopic surgical procedure in an abdominal cavity of a patient, comprising the steps of:
   a) providing a surgical access system including: a gas delivery device, a Veress needle, a valve sealed cannula cap, a gas sealed cannula cap having an obturator connected thereto, a first robotic cannula, a second robotic cannula and a filtered tube set that includes a filter cartridge, a single lumen tube portion and a dual lumen tube portion, wherein a plug is attached to a fitting at the distal end of the dual lumen tube portion of the filtered tube set;
   b) inserting the filter cartridge of the filtered tube set into a reception port of the gas delivery device;
   c) locking the filter cartridge within the reception port by rotating a mechanical lever arm of the gas delivery device;
   d) confirming that a flow setting and a pressure setting on the gas delivery device are appropriate for the patient;
   e) inserting the Veress needle into the abdominal cavity of the patient;
   f) connecting the single lumen tube portion of the filtered tube set to a connector of the Veress needle;
   g) insufflating the abdominal cavity though the Veress needle until a set abdominal pressure is reached;
   h) connecting the gas sealed cannula cap to the second robotic cannula together with the obturator;
   i) inserting the first robotic cannula into the abdominal cavity of the patient with the valve sealed cannula cap connected thereto;
   j) inserting the second robotic cannula into the abdominal cavity of the patient with the gas sealed cannula cap connected thereto;
   k) removing the plug from the fitting at the distal end of the dual lumen tube portion of the filtered tube set;
   l) connecting the fitting of the dual lumen tube portion of the filtered tube set to a connector of the gas sealed cannula cap;
   m) disconnecting the single lumen tube portion of the tube set from the Veress needle;
   n) connecting the single lumen tube portion of the tube set to a connector of the valve sealed cannula cap, whereupon the gas delivery device will begin circulating pressurized gas through the gas sealed cannula by way of the dual lumen portion of the tube set and the gas delivery device will run a calibration process; and
   o) removing the obturator from the gas sealed cannula cap.
34. The method of Clause 33, wherein upon the conclusion of the robotically assisted laparoscopic surgical procedure, further comprising the steps of:
   a) replacing the obturator in the gas sealed cannula cap;
   b) stopping the circulation of pressurized gas by the gas delivery device; and
   c) disconnecting the dual lumen portion of the tube set from the connector of the gas sealed cannula cap.

## Claims

1. A gas sealed access cap for a robotic cannula comprising:
a) a housing having a lid defining a central access port extending to an interior cavity supporting an annular jet assembly for receiving pressurized gas from an inlet port of the housing, wherein the annular jet assembly includes a central aperture aligned with the central access port of the lid and is adapted to generate a gaseous sealing zone within the robotic cannula to maintain a stable pressure within the surgical cavity of a patient;
b) a sound attenuating disc positioned within the housing between the lid and the annular jet assembly, coaxial with the central access port of the lid for reducing noise resulting from generating the gaseous sealing zone;
c) a plurality of circumferentially spaced apart radially inwardly extending vanes formed integral with the housing and located within the interior cavity thereof below the annular jet assembly for directing spent gas from the gaseous sealing zone to an outlet port of the housing;
d) a central access tube aligned with the central access port of the lid and the central aperture of the annular jet assembly, and extending distally from the interior cavity of the housing below the vanes for communicating with a tubular portion of the robotic cannula, wherein a distal end of the housing extends distally beyond a distal end of the central access tube for reception within a proximal bowl portion of the robotic cannula;
e) a pair of diametrically opposed flexible clips extending from a circumferential flange that is integrally formed with an exterior surface of the housing for releasably securing the access cap to the proximal bowl portion of the robotic cannula; and
f) an obturator having a proximal handle portion for cooperatively engaging with the lid of the housing and an elongated obturator shaft extending distally from the handle and having a distal cutting tip, wherein an annular seal is supported on the obturator shaft at a location along the length thereof for sealing against an interior surface of the central access tube adjacent the distal end thereof when the obturator shaft is extended through the central access port of the housing.

2. A gas sealed access cap as recited in Claim 1, wherein an O-ring seal is seated in a circumferential groove formed in the exterior surface of the housing distal to the opposed flexible clips for sealing against an interior surface of the proximal bowl portion of the robotic cannula.

3. A gas sealed access cap as recited in Claim 1, wherein the proximal handle portion of the obturator includes a pair of diametrically opposed spring-biased clips for cooperatively engaging with the lid of the housing.

4. A gas sealed access cap as recited in Claim 1, wherein the distal cutting tip of the obturator is a transparent distal optical cutting tip.

5. A gas sealed access cap as recited in Claim 4 , wherein the proximal handle portion of the obturator defines a central port communicating with a bore that extends through the elongated obturator shaft to the distal optical cutting tip for receiving a scope.

6. A gas sealed access cap as recited in Claim 1, wherein the inlet port of the housing and the outlet port of the housing communicate with a manifold associated with a bullseye connector fitting for communicating with a pressurized gas line and a return gas line of a filtered tube set.

7. A gas sealed access cap as recited in Clam 1, wherein the sound attenuating disc is made from a foam material.
